Europäisches Patentamt

European Patent Office

Office européen des brevets

(19)

(11) Veröffentlichungsnummer : **0 489 043 B1**

(12) ## EUROPÄISCHE PATENTSCHRIFT

(45) Veröffentlichungstag der Patentschrift :
02.02.94 Patentblatt 94/05

(51) Int. Cl.$^5$ : **A61K 31/275**

(21) Anmeldenummer : **90912145.1**

(22) Anmeldetag : **22.08.90**

(86) Internationale Anmeldenummer :
**PCT/EP90/01398**

(87) Internationale Veröffentlichungsnummer :
**WO 91/02524 07.03.91 Gazette 91/06**

(54) **VERWENDUNG DES (+)-ENANTIOMEREN VON ANIPAMIL.**

(30) Priorität : **26.08.89 DE 3928287**

(43) Veröffentlichungstag der Anmeldung :
**10.06.92 Patentblatt 92/24**

(45) Bekanntmachung des Hinweises auf die
Patenterteilung :
**02.02.94 Patentblatt 94/05**

(84) Benannte Vertragsstaaten :
**AT BE CH DE FR GB IT LI NL**

(56) Entgegenhaltungen :
**EP-A- 0 270 782**
**EP-A- 0 276 369**

(73) Patentinhaber : **KNOLL AG**
**Postfach 21 08 05**
**D-67061 Ludwigshafen (DE)**

(72) Erfinder : **MUELLER, Claus, D.**
**Im Schaflaeger 30**
**D-6806 Viernheim (DE)**
Erfinder : **GRIES, Josef**
**Roemerweg 43**
**D-6706 Wachenheim (DE)**

(74) Vertreter : **Karau, Wolfgang, Dr. et al**
**BASF Aktiengesellschaft, Patentabteilung**
**ZDX - C 6**
**D-67056 Ludwigshafen (DE)**

**Beschreibung**

Die Erfindung betrifft eine neue Verwendung des (+)-Enantiomeren von Anipamil (= 1,7-Bis-(3-methoxyphenyl)-3-methylaza-7-cyan-nonadekan).

Anipamil ist aus der EP-OS 64158 bekannt. Dort ist angegeben, daß sich diese Verbindungen zur Behandlung von funktionellen Erkrankungen des Herz-Kreislaufsystems von Kardiomyopathien und Angiopathien eignet. Weitere Indikationen für die Substanz sind hypoxische bzw. ischämische Erkrankungen, nichtkoronarogene Myokardschädigungen, hoher Blutdruck, Durchblutungsstörungen, Spasmen, Ulcus und allergische Reaktionen.

Darüber hinaus ist aus der US-PS 4 777 183 die Eignung von Anipamil zur Herstellung von Arzneimitteln mit antiarteriosklerotischen Eigenschaften zur Behandlung von Arteriosklerose bekannt.

Es wurde jetzt gefunden, daß das (+)-Enantiomere von Anipamil sehr gute Wirkung gegen Arteriosklerose besitzt.

Gegenstand der Erfindung ist die Verwendung des (+)-Enantiomeren von Anipamil und dessen Salzen mit physiologisch verträglichen Säuren zur Herstellung von Arzneimitteln mit antiarteriosklerotischer Wirkung.

Arteriosklerose ist eine häufig auftretende, auf multifaktoriellen Ursachen beruhende Erkrankung der arteriellen Gefäßwand in deren Folge es durch Bildung von Atheromen zur Einengung der Strombahn und zu Gefäßverschlüssen kommt. Im weiteren Verlauf der Krankheit treten häufig Endothelrupturen mit Blutungen in die Plaque und nachfolgende Thrombosierung des betroffenen Gefäßabschnittes (Myocardinfarkt, Schlaganfall) auf.

Mit der erfindungsgemäßen Verbindung kann die Bildung von Atheromen in der Gefäßwand und das Fortschreiten bereits bestehender arteriosklerotischer Veränderungen gehemmt bzw. deren Regression gefördert werden.

Zum Nachweis der antiarteriosklerotischen Wirkung wird das (+)-Enantiomere von Anipamil männlichen Kaninchen über 10 Wochen täglich oral appliziert. Zur Erzeugung von Arteriosklerose erhalten die Tiere eine Diät die 2 % Cholesterin enthält. Am Ende der Versuchsperiode werden die Tiere getötet. Die Aorta wird zwischen den Aortenklappen und der Bifurcatio aortea entnommen, entlang der Medianlinie eröffnet und mit Sudan IV gefärbt. Die sich rot färbenden arteriosklerotisch veränderten Flächen der Intima werden planimetrisch bestimmt. Das Ausmaß der antiarteriosklerotischen Wirkung wird durch Vergleich mit unbehandelten Kontrolltieren festgestellt. Als Vergleichssubstanz dient racemisches Anipamil.

Das (+)-Enantiomere von Anipamil hemmt die Entwicklung von Arteriosklerose im gleichen Ausmaß wie Anipamil (Tab. 1). Zur Behandlung von Arteriosklerose ist die erfindungsgemäße Verbindung dem Anipamil aber deutlich überlegen, da sie in gleicher oder wesentlich höherer Dosierung über keine kardiovaskulären Nebenwirkungen wie beispielsweise blutdrucksenkende Wirkung (Tab. 2) verfügt.

Tabelle 1: Antiatherogene Wirkung am Kaninchen
Tagesdosis: 30 mg/kg per os

| Substanz | Tier-zahl | Aortenfläche | | |
|---|---|---|---|---|
| | | Gesamt-fläche $cm^2$ | atheromatös veränderte Fläche $cm^2$ | Änderung % |
| Kontrollgruppe | 8 | $19\pm0,66$[1] | $16\pm0,91$ | – |
| (+)-Enantiomeren von Anipamil | 10 | $20\pm0,78$ | $12\pm0,12$* | $27\pm7,0$ |
| Anipamil | | $20\pm0,89$ | $11\pm0,12$* | $34\pm6,4$ |

[1] $\bar{x} \pm \bar{sx}$
* $p < 0,01$

2

```
Tabelle 2:  Wirkung auf den Blutdruck wacher spontan hypertensiver Ratten
            (SHR), Applikation per os
```

| Substanz | ED 20 % (mg/kg)[1] Zeit post applicationem | Blutdrucksenkung | |
|---|---|---|---|
| | 2 h | 6 h | 24 h |
| (+)-Enantiomeren von Anipamil | > 100 | > 100 | > 100 |
| Anipamil | 15,8 | 15,4 | 37,4 |

[1] Dosis, welche den Blutdruck um 20 % senkt.

Das (+)-Enantiomere von Anipamil bzw. dessen Salze können oral oder parenteral verabreicht werden. Die Dosierung hängt vom Alter, Zustand und Gewicht des Patienten sowie von der Applikationsart ab. In der Regel beträgt die tägliche Wirkstoffdosis etwa 1 bis 30, vorzugsweise 5 bis 25 mg/kg Körpergehalt bei oraler oder rektaler Gabe und 0,05 bis 5, vorzugsweise 0,5 bis 3 mg/kg Körpergehalt bei parenteraler Gabe.

Gegebenenfalls wird das (+)-Enantiomere von Anipamil in das Säureadditionssalz einer physiologisch verträglichen Säure überführt. Eine Zusammenstellung üblicher physiologisch verträglicher Säuren kann aus "Fortschritte der Arzneimittelforschung", 1966, Deutschland, Schweiz, Birkhäuser Verlag, Bd. 10, S. 224 bis 285 und J. Pharm. Sci., Bd. 66 (1977), S. 1 bis 5 entnommen werden. Bevorzugt ist Salzsäure.

Das Säureadditionssalz wird in der Regel in an sich bekannter Weise durch Mischen der freien Base oder deren Lösung mit der entsprechenden Säure oder deren Lösungen in einem organischen Lösungsmittel, beispielsweise einem niederen Alkohol, wie Methanol, Ethanol oder Propanol, oder einem niedrigen Keton, wie Aceton, Methylethylketon oder Methylisobutylketon, oder einem Ester, wie Diethylester, Tetrahydrofuran oder Dioxan erhalten. Zur besseren Kristallabscheidung können Mischungen der genannten Lösungsmittel verwendet werden. Darüber hinaus können pharmazeutisch vertretbare wäßrige Lösungen von Säure-Additionsverbindungen des (+)-Anipamils durch Auflösen der freien Base in einer wäßrigen Säurelösung hergestellt werden.

Das (+)-Enantiomere von Anipamil und dessen Salze können in den gebäuchlichen galenischen Applikationsformen fest oder flüssig angewendet werden, z.B. in Form von Tabletten, Filmtabletten, Kapseln, Pulvern, Granulaten, Dragees, Suppositorien, Lösungen oder Dosieraerosolen. Diese werden in der üblichen Weise hergestellt. Die Wirkstoffe können dabei mit den gebräuchlichen galenischen Hilfsmitteln wie Tablettenbindern, Füllstoffen, Konservierungsmitteln, Tablettensprengmitteln, Fließregulierungsmitteln, Weichmachern, Netzmitteln, Dispergiermitteln, Emulgatoren, Lösungsmitteln, Retardierungsmitteln und/oder Antioxidantien verabreicht werden (vgl. H. Sucker et al.: Pharmazeutische Tecknologie, Thieme-Verlag, Stuttgart (1978). Die so erhaltenen Zubereitungen enthalten den Wirkstoff normalerweise in einer Menge von 0,1 bis 99 Gew.-%.

Beispiel 1

Auf einer Tablettenpresse werden in üblicher Weise Tabletten folgender Zusammensetzung gepreßt:

| | |
|---|---|
| 80 mg | (+)-Enantiomeres von Anipamil |
| 235 mg | Maisstärke |
| 27 mg | Gelatine |
| 90 mg | Milchzucker |
| 4,5 mg | Aerosil® (chemisch reine Kieselsäure in submikroskopisch feiner Verteilung) |
| 13,5 mg | Kartoffelstärke (als 6 %iger Kleister) |

Beispiel 2

In üblicher Weise werden Dragees folgender Zusammensetung hergestellt:

| | |
|---|---|
| 40 mg | (+)-Enantiomeres von Anipamil |
| 120 mg | Kernmasse |
| 120 mg | Verzuckerungsmasse |

Die Kernmasse besteht aus 9 Teilen Maisstärke, 3 Teilen Milchzucker und 1 Teil Vinylpyrrolidon-Vinylacetat-Mischpolymerisat 60:40 (vgl. Pharm. Ind. 1962, 586). Die Verzuckerungsmasse besteht aus 5 Teilen Rohrzucker, 2 Teilen Maisstärke, 2 Teilen Calciumcarbonat und 1 Teil Talk. Die so hergestellten Dragees werden anschließend mit einem magensaftresistenten überzug versehen.

Beispiel 3

10 g (+)-Enantiomeres von Anipamil werden in 5000 ml Wasser unter Zusatz von NaCl gelöst und auf pH 6,0 eingestellt, so daß eine blutisotonische Lösung entsteht.

**Patentansprüche**

1. Verwendung des (+)-Enantiomeren von Anipamil und dessen Salzen mit physiologisch verträglichen Säuren zur Herstellung von Arzneimitteln mit antiarteriosklerotischer Wirkung.

**Claims**

1. The use of the (+) enantiomer of anipamil and its salts with physiologically tolerated acids for the production of pharmaceuticals with antiarteriosclerotic action.

**Revendications**

1. Utilisation de l'énantiomère-(+) de l'anipamil et de ses sels avec des acides acceptables physiologiquement pour la préparation de médicaments à action antiartériosclérotique.